(19) Europäisches Patentamt
European Patent Office
Office européen des brevets

(11) **EP 2 548 026 B1**

(12) **EUROPEAN PATENT SPECIFICATION**

(45) Date of publication and mention of the grant of the patent:
**04.11.2015 Bulletin 2015/45**

(51) Int Cl.:
***G01N 33/569*** (2006.01)

(21) Application number: **11714085.5**

(22) Date of filing: **15.03.2011**

(86) International application number:
**PCT/GB2011/050511**

(87) International publication number:
**WO 2011/114145 (22.09.2011 Gazette 2011/38)**

(54) **DETECTION OF OVERTRAINING SYNDROME IN AN INDIVIDUAL**

NACHWEIS VON ÜBERTRAININGSSYNDROM BEI EINER PERSON

DÉTECTION DU SYNDROME DE SURENTRAÎNEMENT CHEZ UN INDIVIDU

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.03.2010 GB 201004239**

(43) Date of publication of application:
**23.01.2013 Bulletin 2013/04**

(73) Proprietor: **Knight Scientific Limited Plymouth, Devon PL2 3BY (GB)**

(72) Inventor: **KNIGHT, Jan Plymouth Devon PL2 3BY (GB)**

(74) Representative: **Wilkinson, Stephen John Stevens Hewlett & Perkins 1 St Augustine's Place Bristol BS1 4UD (GB)**

(56) References cited:
- KNIGHT JAN ET AL: "Luminescent reactions of Pholasin with isolated human leucocytes and diluted whole blood", EUROPEAN JOURNAL OF CLINICAL INVESTIGATION, WILEY-BLACKWELL PUBLISHING LTD, GB, vol. 26, no. suppl. 1, 1 January 1996 (1996-01-01), page A37, XP009147998, ISSN: 0014-2972
- PAVELKOVA MARTINA ET AL: "Luminol-, isoluminol- and lucigenin-enhanced chemiluminescence of rat blood phagocytes stimulated with different activators.", LUMINESCENCE (CHICHESTER), vol. 19, no. 1, January 2004 (2004-01), pages 37-42, XP002636323, ISSN: 1522-7235
- REICHL S ET AL: "The photoprotein Pholasin as a luminescence substrate for detection of superoxide anion radicals and myeloperoxidase activity in stimulated neutrophils", FREE RADICAL RESEARCH, YVERDON, CH, vol. 35, no. 6, 1 January 2001 (2001-01-01), pages 723-733, XP009148036, ISSN: 1071-5762
- RAIDAL S L ET AL: "Effects of training on resting peripheral blood and BAL-derived leucocyte function in horses", EQUINE VETERINARY JOURNAL, vol. 33, no. 3, May 2001 (2001-05), pages 238-243, XP002636324, ISSN: 0425-1644
- CHINDA DAISUKE ET AL: "A competitive marathon race decreases neutrophil functions in athletes.", LUMINESCENCE (CHICHESTER), vol. 18, no. 6, November 2003 (2003-11), pages 324-329, XP002636325, ISSN: 1522-7235
- TIIDUS PETER M: "Radical species in inflammation and overtraining", CANADIAN JOURNAL OF PHYSIOLOGY AND PHARMACOLOGY, vol. 76, no. 5, May 1998 (1998-05), pages 533-538, XP002636326, ISSN: 0008-4212
- LI HAI-TAO ET AL: "Two-peak kinetic curve of the chemiluminescence in phorbol-induced macrophage", BIOCHEMICAL AND BIOPHYSICAL RESEARCH COMMUNICATIONS, vol. 223, no. 2, 1996, pages 311-314, XP002636327, ISSN: 0006-291X

**Description**

The present invention relates to a method for detecting the onset, or the existence, of the condition known as overtraining syndrome in a human or non-human mammal. More particularly, it relates to a method by which overtraining syndrome in an athlete may be identified.

It is normal for an individual (i.e. a human or non-human mammal) to suffer from tiredness after a session of strenuous physical exercise and for the individual to recover after a period of rest. Many individuals engage in regular and prolonged training programmes involving intense physical activity, particularly individuals training to participate in a sports event or competition. Successful athletes, whether they are recreational athletes or professional or elite athletes, apply the fundamental principle of athletic training which involves workouts followed by rest. As the workout becomes longer and more intense, the effectiveness of the rest period that follows similarly has to increase. In such a cycle of workout followed by rest, it is during the rest periods that the body repairs itself and becomes stronger, thus providing for subsequent improvement in performance. An individual who does not maintain a correct balance between workouts and rest does not allow his or her body to recover (described as "overreaching" in the short term) such that, for instance, depleted energy stores do not become fully replenished and muscle tissue damage does not become fully repaired. Overtraining syndrome is a condition which can develop in an athlete who fails to maintain a sufficient balance between workouts and recovery time and is, in essence, "under recovered". Overtraining syndrome, also referred to as "staleness" or "burnout" by athletes, presents symptoms such as excessive fatigue, exhaustion, decreased performance levels and higher or lower than expected heart rates at set intensities. In addition, overtraining syndrome is often associated with depression, irritability, disturbed sleep patterns, decreased appetite, changes in hormone levels, including testosterone and cortisol, immunosuppression leading to increased vulnerability to infections, and general malaise. Depending on the degree to which the athlete is overtrained, it can take upwards of six weeks and, possibly, up to six months for the athlete to recover.

While the syndrome most commonly affects athletes at the high performance, elite level and even Olympic level, overtraining can affect athletes at any level and in any sport.

It is also the case that certain non-human mammals that are exercised and trained to perform in racing competition events suffer from overtraining. Examples of such non-human mammals include horses, dogs (including solo runners such as greyhounds and sleddogs such as huskies and Eurohounds), and camels.

Currently, there is no diagnostic test available for overtraining syndrome. Athletes suffering from chronic fatigue and long term underperformance may undergo clinical investigation, the purpose of which is to exclude other and potentially more serious causes for chronic fatigue.

We have discovered that, surprisingly, the onset and occurrence of overtraining syndrome in an individual can be detected by a quick testing procedure which can be carried out using a very small sample of blood provided by the individual.

Summary of the invention

The present invention provides a method for detecting the onset of or the existence of overtraining syndrome in an individual, which method comprises

> a) contacting leucocytes in, or obtained from, a blood sample provided by the individual with a luminescence reagent which emits light on reaction with an oxidant;
> b) adding an activator to the mixture of leucocytes and the luminescence reagent to stimulate the NADPH oxidase system of the leucocytes, wherein the activator is selected from N-formyl-methionyl-leucyl-phenylalanine and platelet activating factor;
> c) continuously monitoring and/or measuring light emitted by the luminescence reagent over a predetermined time period commencing before and ending after the addition of the activator, whereby a light emission curve is produced showing the light emitted by the luminescence reagent over the predetermined time period;
> d) assessing the features of the light emission curve for determining onset of or existence of overtraining syndrome in the individual by comparison with one or more standard signature curves and the determining is based on the similarity or dissimilarity occurring between features of the light emission curve and features of the one or more standard signature curves.

The method of detecting the onset of or the existence of overtraining syndrome, in accordance with the invention, is mainly concerned with human athletes and/or sports performers (both solo performers and sports team members) although it may also be useful for human performers in other fields whose performance demands regular and rigorous physical training, such as professional dancers and professional ice skaters. Although the word "individual", as used above, is principally intended to refer to human individuals, it does, however, also cover non-human mammals that are typically bred, exercised and trained to perform in racing competition events. Examples of such animals include horses, particularly racehorses, dogs (both solo runners such as greyhounds and team performers such as sleddogs, e.g. huskies and Eurohounds), and camels.

Although the invention in its broadest scope includes the detection of overtraining syndrome in non-human mammals as stated above, it will be discussed further in connection with its application to human individuals.

In step c) of the method of the invention, a light emission curve is produced showing the light emitted by the luminescence reagent over the predetermined time period. We have found that certain features of the light emission curve obtained for an individual known to be suffering from overtraining syndrome differ from those of a light emission curve obtained for a normal, fit, healthy person and that, therefore, the features of the light emission curve obtained for an individual under test can be assessed in order to determine onset of or occurrence of overtraining syndrome. According to step d), the assessment of the features of the light emission curve is carried out by comparing the light emission curve with one or more standard signature curves. The determination is based on the similarity or dissimilarity occurring between one or more features of the light emission curve obtained for the individual under test and features of the one or more standard signature curves.

Detailed description of the invention

The method for detecting the onset of or the existence of overtraining syndrome in an individual, typically an athlete, comprises

> a) contacting leucocytes in, or obtained from, a blood sample provided by the individual with a luminescence reagent which emits light on reaction with an oxidant;
> b) adding an activator to the mixture of leucocytes and the luminescence reagent to stimulate the NADPH oxidase system of the leucocytes, wherein the activator is selected from N-formyl-methionyl-leucyl-phenylalanine and platelet activating factor;
> c) continuously monitoring and/or measuring light emitted by the luminescence reagent over a predetermined time period commencing before and ending after the addition of the activator, whereby a light emission curve is produced showing the light emitted by the luminescence reagent over the predetermined time period;
> d) assessing the features of the light emission curve for determining onset of or existence of overtraining syndrome in the individual by comparison with one or more standard signature curves and the determining is based on the similarity or dissimilarity occurring between features of the light emission curve and features of the one or more standard signature curves.

Overtraining syndrome is defined as a condition, suffered by an athlete, of fatigue and underperformance often associated with other and varied symptoms, such as frequent infections, depression and sleep deprivation, which occurs following hard training and competition when there is no other identifiable medical cause. Any athlete, in any sport, may experience overtraining syndrome if he or she is engaged in long and heavy training programmes which do not allow adequate recovery time between each training session. The problem is more likely to occur in high performance or elite-level athletes because these athletes are driven towards achieving success in competitions at world class level and, as such, may ignore early warning signs of overtraining in the pursuit of their competitive goal. The method of the invention provides an early screening procedure that may be carried out on an individual, before the individual may present severe symptoms that require detailed and expensive medical investigation. The method allows, through a simple technique using a blood sample provided by an individual, the detection of early signs of overtraining which may be detected even before the individual is aware that he or she has any symptoms. The method of the invention, therefore, provides the opportunity for athletes, and their coaches, to monitor training effects and performance such that early intervention may be made to the athletes' training schedules before the onset of serious and long term symptoms.

The method of the invention is carried out on leucocytes contained in, or obtained from, a blood sample taken from the individual under test. The test can be carried out using whole blood (optionally diluted) or on a leucocyte-containing isolate, such as isolated leucocytes. Methods of isolating leucocytes from whole blood are, of course, well known. Only a small sample is required for testing in the method, such as provided from a pin prick device or lancet rendered to the individual in order to obtain capillary blood. However, the sample can also be obtained from venous blood collected in the normal way. The blood should be collected with an anticoagulant present in order to prevent the normal clotting process but if used immediately, a small amount of blood can be diluted with assay buffer.

According to the method of the invention, the blood sample, typically diluted, or a sample of leucocytes isolated from whole blood, is contacted with a luminescence reagent which emits light on reaction with an oxidant. Such luminescence reagents, generally, are well-known in the art and examples include lucigenin, luminol (3-aminophthalhydrazide, 5-amino-2,3-dihydro-1,4-phthalazinedone), isoluminol (4-aminophthalhydrazide), MCLA (6-(methoxyphenyl)-2-methyl-3,7-dihydroimidazol[1,2-a]pyrazine-3(7H)-one hydrochloride and PHOLASIN (™) (PHOLASIN is a registered trade mark of Knight Scientific Limited) which is the photoprotein derived from the marine bivalve mollusc *Pholas dactylus.* PHOLASIN (™) is preferred for use in the present invention in view of its ultrasensitity towards free radicals such as the superoxide anion and other reactive oxygen-containing species (ROS) as well as its ability to react with enzymes such as peroxidases. It is also possible to use a synthetic equivalent of the photoprotein derived from *Pholas dactylus.*

An activator is added to the mixture of leucocytes and luminescence reagent to stimulate the NADPH oxidase system of the leucocytes. Through this stimulation, free radicals and/or reactive oxygen species produced excite the luminescence reagent resulting in the emission of light by the reagent. Examples of activators that may be used in the method of the present invention include the receptor stimulant N-formyl-methionyl-leucyl-phenylalanine (fMLP). Presentation of fMLP and the phorbol ester, phorbol 12-myristate-13-acetate (PMA), which enters the cell and activates protein kinase C directly, together enables the activation of the NADPH oxidase on the cell surface to be monitored simultaneously with the activation of the NADPH oxidase on the membrane of secondary granules. PMA activates the NADPH oxidase throughout the cell, but at a slower rate than fMLP and it also promotes degranulation. In addition, platelet activating factor (PAF), which binds to fMLP receptors and also enters the cell can also be used since it acts in a similar way to using a combination of fMLP and PMA. Other mediators, such as anti-Fc receptor antibodies, activated complement and lipopolysaccharides (LPS) may also be used in concentrations that either prime the cell to respond to fMLP or at higher concentrations to actually stimulate the production of free radicals. These mediators, in addition to stimulating the production of free radicals, can also promote the release of enzymes from various granules within the leucocytes.

According to the method of the invention, light that is emitted by the luminescence reagent is monitored and/or measured over a predetermined time period which commences before the addition of the activator to the mixture of luminescence reagent and leucocytes and which ends after the addition of the activator. The light emitted will typically be monitored and/or measured by the use of a luminometer. In the case of the luminescence reagent PHOLASIN (™), a low level light, known as the resting glow, is emitted before the leucocytes are activated but, on the addition of the activator, light emission is increased to a level determined by the degree of stimulation of the leucocytes and the light emitted over the time period of the observation changes with time elapsed since the addition of the activator. Thus, over the predetermined time period during which light emission is monitored and/or measured, the effect of the addition of the activator to the leucocyte/luminescence reagent mixture, i.e. the level of activation of the leucocytes, is seen. The predetermined time period will be chosen depending on the concentration of leucocytes and/or luminescence reagent in the test solution but will typically be less than 30 minutes, preferably less than 20 and more preferably less than 15 minutes.

The emission of light by the luminescence reagent is recorded with respect to time over the predetermined period of time. Through our research we have found, surprisingly and unexpectedly, that a plot of emitted light intensity against time produces a light emission curve which, in the case of an individual affected by overtraining syndrome, is characteristically different from a control light emission curve produced using leucocytes taken from a normal, fit, healthy individual. We have found, for instance, that the peak intensity of emitted light recorded after the addition of the activator to the mixture of leucocytes and luminescence reagent and/or the overall shape of the light emission curve, compared to a control light emission curve, may be indicative of one or more problems in the health and wellbeing of the individual under test. A control, light emission curve produced using leucocytes obtained from a blood sample taken from a normal, fit and healthy individual has a shape characterised by an immediate and steep rise in light emission, following the addition of the activator, to reach a peak, which peak is followed by a significant tailing off of light emission with time elapsed from the peak value, gradually returning to low light emission within a few minutes. Contrariwise, a light emission curve produced using leucocytes obtained from a blood sample taken from a fatigued individual is, compared to the control, blunted and characterised by a peak value significantly lower than the peak value shown in the control curve. Furthermore, the light emission curve obtained for such an individual typically shows a rate of increase in emitted light, following the addition of the activator to the leucocyte/luminescence reagent mixture, which is lower than that observed in the control curve (i.e. the rise is less steep) and the peak value reached in the light emission is followed by a much more gradual decrease in light emission over time, as compared to the control curve, and which in many cases an almost inverse linear relationship between light emitted and time elapsed is observed. The differences between the light emission curve obtained for such a fatigued individual and the control curve become more pronounced as the level of fatigue or exhaustion experienced by the individual under test increases.

Compared to the control curve, a curve produced using leucocytes obtained from a blood sample taken from an individual who is infected by an infection agent, whether or not the individual has experienced any symptoms of the infection at the time the blood sample is taken, shows an excessive response characterised by a peak light emission value substantially higher than that of the control. In the very early stages of an infection in the individual, the peak of the light emission curve obtained is sharper than the peak shown in the light emission curve obtained for the individual at a later stage in the infection. As the infection proceeds, the general shape of the curve becomes more rounded compared to the curve obtained in the early stages of the infection. Accordingly, a characteristically shaped curve, produced presymptomatically, is useful for informing the individual that he or she may feel unwell and may present symptoms of an infection within a finite period of time, for instance within 1 or 2 days, following the time of providing the blood sample.

It is, according to the present invention, possible to produce a library of signature light emission curves which may be used as standards against which a light emission curve produced using leucocytes obtained from a blood sample taken from an individual under test may be compared. Using such a comparison against such standard signature curves, it is possible not only to determine whether or not the light emission response obtained for an individual under test is normal or abnormal but also, if the light emission response is not normal compared to a control, to identify a possible reason for the abnormality. Individuals who, according to the results obtained by the method of the invention, show an abnormal response may then be selected to undergo more detailed medical testing to identify problems. This would almost always be the case if the light emission curve indicates the onset of fatigue or indicates presymptomatically the presence of an infection agent. When a fatigued athlete succumbs to an infection, a high intensity light response may be seen superimposed upon a depressed response giving further clues to an additional problem.

According to the invention, the light emission curve produced for an individual under test is compared with one or more standard signature curves and the determination is based on the similarity or dissimilarity occurring between features of the light emission curve and features of the one or more standard signature curves. Preferably, the features of the light emission curve and the features of the one or more standard signature curves are selected from curve shape, intensity of curve and a combination thereof. Typically, the one or more standard signature curves with which the light emission curve produced for an individual under test will be compared, according to the invention, will be selected from curves produced using leucocytes obtained from blood samples of athletes clinically confirmed as suffering from excessive or persistent fatigue, curves produced using leucocytes obtained from blood samples of athletes suffering from poor performance in competition, curves produced using leucocytes obtained from blood samples of athletes unable to sustain normal training levels, curves produced using leucocytes obtained from blood samples of individuals suffering from a viral or bacterial infection and curves produced using leucocytes obtained from blood samples of normal, fit, healthy individuals. The curves obtained using leucocytes obtained from blood samples of individuals suffering from a viral or bacterial infection will preferably include curves obtained using leucocytes from individuals suffering from viral infections of the respiratory tract since athletes undergoing heavy training regimes seem to be especially vulnerable to infections such as colds and influenza. However, clues to the existence of a bacterial infection, a serious complication of respiratory tract infections, can be seen by a characteristic rounded and broad light emission curve.

A diagnostic kit for use in carrying out the method of the invention may comprise:

> a) a luminescence reagent which emits light on reaction with an oxidant;
> b) an activator; and
> c) a library of standard signature curves.

Preferably, the luminescence reagent provided in the diagnostic kit is the photoprotein derived from *Pholas dactylus* (PHOLASIN (™)). Preferably, the activator provided in the diagnostic kit of the invention is selected from N-formyl-methionyl-leucyl-phenylalanine (fMLP), platelet activating factor, phorbol-12-myristate-13-acetate and lipopolysaccharide.

The library of standard signature curves may be provided on any suitable format, for example, printed on a substrate such as paper, card or a fabric or provided in computer-readable form, such as a disk or other storage device.

The shapes of the light emission curves obtained according to the present invention are dependent on the response of the leucocytes, in terms of the production of free radicals and/or other active substances, to activation by an activator. These responses are dependent on the physiological state of the leucocytes in the blood and are thus related to the physiological or medical state of the patients from whom the blood samples were obtained. While such subjective observations of the shapes of the curves are of great value in making diagnoses, a method of quantifying the curves would lead to a more objective determination of irregularities in the leucocytes in the blood and, hence, a more objective diagnosis.

The curves consist of a plot of the response of a luminometer in terms of Relative Light Units (RLU) in three parts. The first part records the light emission before the stimulation of the leucocytes by the activator and may be analysed separately, if at all. The major part of the curve consists of a rise followed by a fall. The whole of this part of the curve can be analysed as one unit or it can be divided into the rising part and the falling part and the two parts analysed separately.

The curves of light emission against time, produced according to the invention, may be used to provide quantitative and/or qualitative data that may, further, assist in assessing whether an individual's response is normal or abnormal and, if the individual's response is considered to be abnormal, in suggesting a possible reason for the abnormal response.

Quantitative data is provided by the maximum light recorded, i.e. the peak value in the curve, which indicates the intensity of the light emission from the luminescence reagent.

Suitable software can be used to derive a cubic expression of the general form $y = ax^3 + bx^2 + cx + d$, where

y = RLU and x = time elapsed (in seconds). By comparing values of a, b, c and d, calculated from different curves, it is possible to make a quantitative, and therefore more objective, comparison of the responses obtained using blood samples from different patients.

Qualitative data, which can be used to suggest a possible reason for an individual's response, may be derived from a study of the shape of the individual's light response curve and an understanding of how the shapes of light response curves, produced by different individuals, vary.

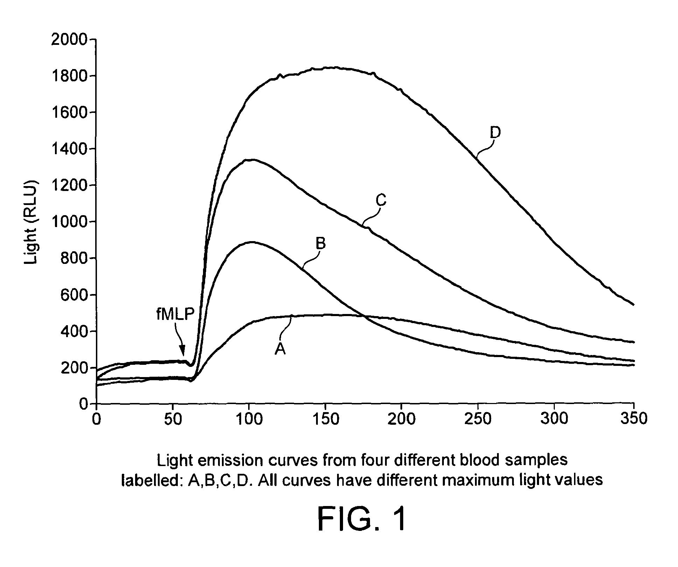

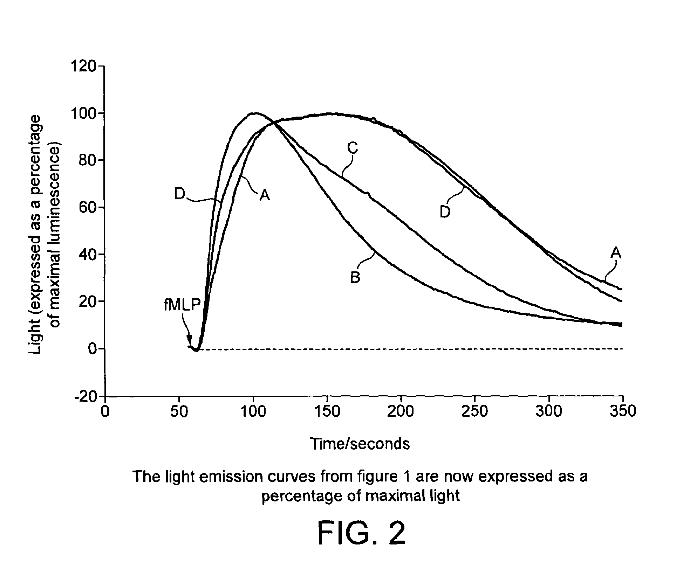

The shapes, rather than the amplitudes, of different curves can be compared according to the method described as follows with reference to Figures 1 and 2.

Figure 1 shows light response curves (light emission (RLU) against elapsed time (seconds)) for four different blood samples (A, B, C and D). In each case, the procedure for preparing the sample and for measuring the light emission was as described in Example 1. The curve for sample A shows a gradual increase in light emission following the addition of the fMLP activator up to a low peak followed by a plateau and gradual decrease in emitted light. Each of the curves for samples B and C shows a steep increase in light emission immediately after the addition of the activator reaching a well-defined peak and, thereafter, a relatively rapid decrease in light emission.

The curve for sample D shows a steep increase in emitted light immediately following the addition of the activator. The curve for sample D shows an intense, but rounded, peak after which the light emission decreases relatively quickly.

In order to compare the shapes of the curves, the following two operations are conducted:

1. In the case of each sample, for each RLU value measured at time t a derived RLU value is obtained by subtracting, from each actual RLU measurement, the initial RLU value measured immediately prior to the addition of the activator, i.e.

$$\text{derived RLU at time } t = \text{actual RLU at time } t \text{ (as per curve in Figure 1)} \quad \text{minus} \quad \text{RLU immediately prior to addition of activator (as per curve in Figure 1)}$$

Thus, for each sample, a set of derived RLU values, over the time period in which emitted light is measured, is obtained.

2. The maximum (i.e. peak) light emission ($RLU_{max}$) for each sample as shown in the curves in Figure 1 is determined.

3. For each sample, each derived RLU value for each time t is expressed as a percentage of the $RLU_{max}$, i.e.

$$\text{Light at time } t \text{ (as percentage of maximum)} = \frac{\text{derived RLU at time } t}{RLU_{max}} \times 100\%$$

The values obtained above for the light at time t are then plotted against time (t).

For each sample, a plot of light at time t against time (t) is shown in Figure 2. The derived curves for samples A and D are seen, in Figure 2, to have very similar shape which was not apparent from the shapes of the curves shown in Figure 1. The derived curves for Samples B and C also have a similar shape but one which is different from the shape of the derived curves for A and D.

As a result of the qualitative analysis described above, with reference to Figures 1 and 2, it is possible to make certain conclusions about the four individuals who provided the blood samples A to D.

The method of the present invention can be carried out using conventional apparatus capable of monitoring and/or measuring light emitted by a luminescence reagent in the presence of activated leucocytes. The method is suitable for being carried out in a hand-held luminometer which is highly portable, compact and simple to use. Such a device comprises a means for containing a mixture of the leucocyte sample to be analysed and the luminescence reagent, a means for introducing, into the mixture, an activator, a means for monitoring and/or measuring light emitted by the luminescence reagent over a period of time commencing before and ending after the introduction of the activator, a display for displaying the light emitted by the luminescence reagent; the device being capable of being gripped in one hand.

EXAMPLES

Example 1

A sterile sample of blood was taken from a male, elite performance athlete who, at the time, felt generally tired and was unable to sustain "normal" training loads. The athlete had a history of repeated illness occurring approximately every two weeks.

The blood sample was collected in a tube containing EDTA and was assayed, as described below, on the day of collection.

2mL of Blood Dilution Buffer were added to an empty tube and, to this, were added $20\mu$L of the EDTA blood to prepare a diluted whole blood sample for testing. The tube was capped and then gently inverted three times to mix the contents of the tube.

To an opaque, white microplate well was added $90\mu$L RECONSTITUTION AND ASSAY BUFFER FOR PHO-LASIN® (Knight Scientific Limited), $20\mu$L reconstituted ADJUVANT-K ("ADJUVANT-K" is a trade mark of Knight Scientific Limited) which is a luminescence enhancer, $50\mu$L PHOLASIN® and $20\mu$L diluted whole blood. The reconstituted ADJUVANT-K luminescence enhancer had previously been prepared from ADJUVANT-K reconstituted with 5mL RECONSTITUTION AND ASSAY BUFFER. The contents of the microplate well were then mixed.

The microplate was then placed in a luminometer, equipped with a shaker, and incubated with shaking at 37°C for 1 minute.

The light emitted from the sample was recorded continually for 1 minute. After this, $20\mu$L of fMLP were injected into the cell and the emission of light was recorded continuously for a further 5 minutes.

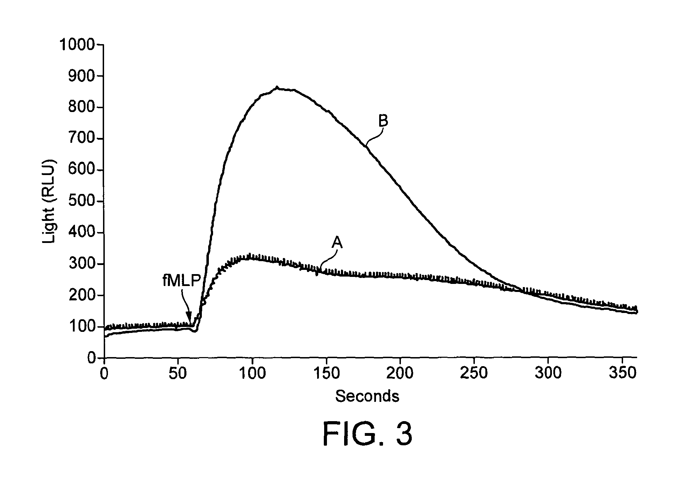

The light emission profile was recorded as a light emission curve showing the light emitted (in relative light units (RLU)) for the period commencing 1 minute before the addition of the fMLP activator and ending 5 minutes after the addition of the activator. Fig.3 shows the light emission curve (A) produced using the blood sample taken from the individual and a control light emission curve (B) produced using a blood sample taken from an individual known to be fit and healthy.

As can be seen from Fig.3, the light response curve obtained using the blood sample under test was stunted compared to the control, showing a peak emission substantially lower than that of the control. The light emitted over the period following the peak decreased very gradually compared to the control.

Example 2

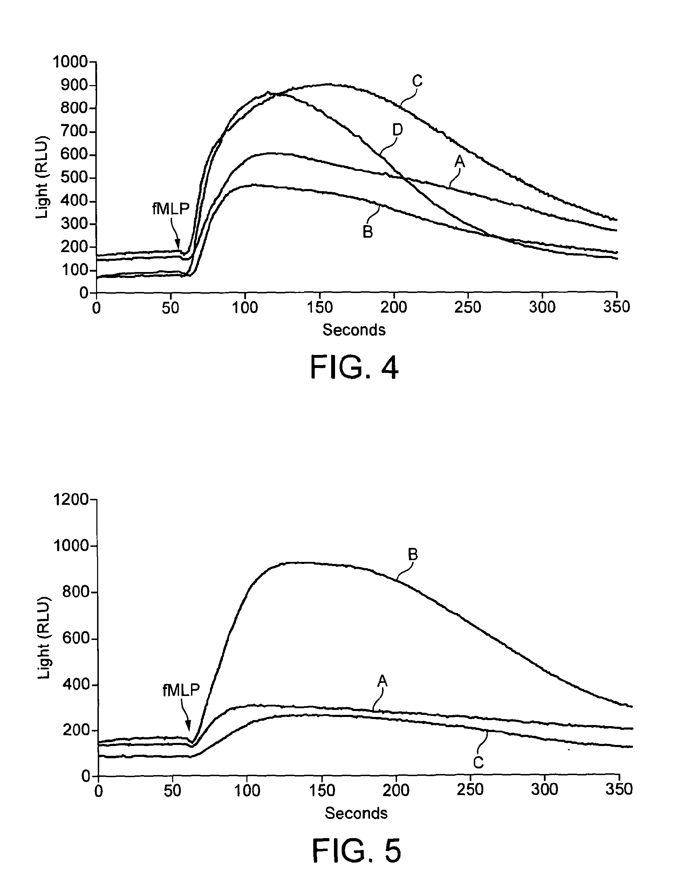

A sample of blood was taken from a performance athlete who reported feeling persistently fatigued. The blood sample was tested, according to the method of the invention, on the day the sample was collected following the procedure described in Example 1. The light emission curve produced using this sample is shown as A in Figure 4. A fresh sample of blood was taken from the same athlete, again reporting a feeling of fatigue, approximately 3 months after the date on which the first sample was collected and tested. The second sample was tested on the day of collection according to the procedure described in Example 1. The light emission curve produced using this second sample is shown as B in Figure 4. The procedure was repeated approximately 5 months after the second sample was collected and tested using a fresh sample of blood provided by the athlete. The light emission curve produced using this third blood sample is shown as C in Figure 4.

Both curves A and B have shapes which are characteristic for individuals suffering from fatigue arising from an imbalance in the cycle of workouts and subsequent recovery periods. Both curves A and B exhibit, compared to the normal control (D), low peak emitted light values, rates of light emitted, following the addition of the activator, before peak values are reached that are lower than that of the control and each exhibits a very gradual decrease in light emitted after the peak value with respect to time which almost conforms to a linear relationship which is very different from the decrease in emitted light over the same period of time shown by the normal control.

Curve C, which was produced using a blood sample taken from the athlete approximately 8 months after the first sample was taken, shows a completely different light emission response. The curve C shows a steep rise in light emission immediately following the addition of the activator which is similar, initially, to that shown in the control curve D. The peak attained by curve C is greater than that attained by the control. Curve C, furthermore, differs from the control curve D by being much more rounded in shape in the lead up to the peak value and in the decreasing level of light emission after the peak value. At the end of the recording period, the light emitted by the third blood sample is still substantially greater than that emitted, at that point in time, by the control.

A subsequent blood film analysis carried out on the third blood sample confirmed that the athlete was, at that time, suffering from an infection.

Example 3

A blood sample was taken from an endurance athlete on a day the athlete was reported to be unable to complete a training session on the running track. The blood sample was prepared and tested according to the procedure described above in Example 1. The light emission curve obtained for this sample is shown as curve A in Figure 5. Eleven days later, the athlete felt recovered and completed a tempo run, i.e. a sustained run at faster than the usual training pace.

The athlete provided a blood sample on that day and this was prepared and tested on the same day according to the procedure described in Example 1. The light emission curve obtained using this blood sample is shown as curve B in Figure 5. 27 days after the first blood sample was taken, the athlete again reported feeling extremely tired and a fresh blood sample was taken on the day and was prepared and tested on the same day according to the procedure described above. The light emission curve obtained using this fresh blood sample is shown as curve C in Figure 5.

As can be seen from Figure 5, the curves A and C have a shape characteristic of a fatigue state: a relative slow increase in light emission following addition of the activator to a suppressed peak value followed by a very gradual decrease in light emission over the remaining period of monitoring. As a contrast to curves A and C, curve B which was produced from a blood sample taken from the athlete at a time the athlete felt recovered and was able to perform well in training has a shape conforming more closely to a standard control, i.e. it demonstrates a steep rise in light emission, following addition of the activator, to a peak substantially higher (compared to curves A and C). After the peak value, the light emission falls off more rapidly (compared to curves A and C) over the remaining period of monitoring.

<u>Example 4</u>

Two elite performance athletes (I and II) provided blood samples for testing. On the day the samples were taken and were tested, both athletes reported well. The blood samples were submitted for full blood count (FBC) analysis and also for testing according to the method of the invention. FBC measures the status of a number of different features of the blood, including the amount of haemoglobin in the blood, the number of red blood cells (red cell count), the percentage of blood cells as a proportion of the total blood volume (haematocrit), the volume of red blood cells (mean cell volume), the average amount of haemoglobin in the red blood cells (mean cell haemoglobin), the number of white blood cells (white cell count), the percentages of the different types of white blood cells (leucocyte differential count); and the number of platelets. The blood samples of both athletes were found to be FBC normal.

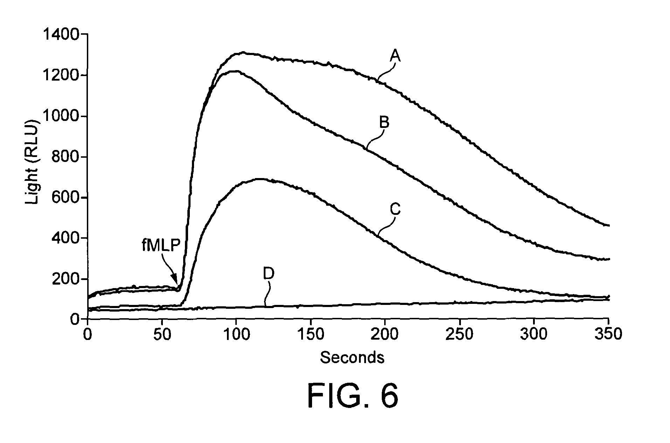

The athletes' samples were prepared and tested according to the method of the invention as described in Example 1 above on the same day the samples were obtained. The light emission curves obtained using these blood samples are shown in Figure 6, where curve A is the light emission curve obtained using the sample taken from athlete I and curve B is the light emission curve obtained using the sample taken from athlete II. Figure 6 also contains two control curves: curve C which is a normal control light emission curve produced, according to the invention, using a blood sample taken from a normal, fit and healthy individual, and curve D which is a plasma control curve produced, according to the invention, using a sample of the plasma ($20\mu$L plasma diluted 1:100 with blood dilution buffer) obtained from the whole blood samples taken from the athletes.

Curve A, compared to the normal control curve C, shows an excessive response following the addition of the activator to the leucocyte/luminescence reagent mixture. It shows an extremely steep increase in light emission, following the addition of the activator, which is steeper than that of the control C. The peak light emission reached in curve A is much greater than that reached in the control curve C. Curve B also shows (like curve A) a very steep increase in light emission, following the addition of the activator, and reaches a peak value lower than that in curve A but still substantially greater than that in the normal control curve C. In curve A, subsequent to reaching the peak emission, the curve almost plateaus for a period of about 100 seconds before decreasing to a light emission value of about 500 RLU at 350 seconds after commencement of monitoring (compared to about 100 RLU for the normal control). In curve B, no plateauing was observed in the light emission following the peak value and, instead, light emission decreases significantly but gradually to an emission value of about 300 RLU 350 seconds after the commencement of monitoring.

Although, on the day of the tests, both athletes I and II reported feeling well, athlete I reported feeling unwell the day after the test and athlete II reported feeling unwell two days after the test. Both athletes were confirmed, after symptoms of illness were experienced, as suffering from infections. The results obtained according to the method of the invention (curves A and B) indicate the presence of predictive markers in the blood of athletes I and II, respectively, and show that the method of the invention has use as a presymptomatic test for illness.

**Claims**

1. A method for detecting the onset of or the existence of overtraining syndrome in an individual, which method comprises

    a) contacting leucocytes in, or obtained from, a blood sample provided by the individual with a luminescence reagent which emits light on reaction with an oxidant;
    b) adding an activator to the mixture of leucocytes and the luminescence reagent to stimulate the NADPH oxidase system of the leucocytes, wherein the activator is selected from N-formyl-methionyl-leucyl-phenylalanine and platelet activating factor;
    c) continuously monitoring and/or measuring light emitted by the luminescence reagent over a predetermined time period commencing before and ending after the addition of the activator, whereby a light emission curve is produced showing the light emitted by the luminescence reagent over the predetermined time period;

d) assessing the features of the light emission curve for determining onset of or existence of overtraining syndrome in the individual by comparison with one or more standard signature curves and the determining is based on the similarity or dissimilarity occurring between features of the light emission curve and features of the one or more standard signature curves.

2. A method according to claim 1, wherein the features of the light emission curve and the features of the one or more standard signature curves are selected from curve shape, intensity of curve and a combination thereof.

3. A method according to either claim 1 or claim 2, wherein the light emission curve is produced using leucocytes in, or obtained from, a blood sample taken from an athlete and wherein standard signature curves are selected from curves produced using leucocytes in, or obtained from, blood samples of athletes clinically confirmed as suffering from excessive or persistent fatigue, curves produced using leucocytes in, or obtained from, blood samples of athletes suffering from poor performance in competition, curves produced using leucocytes in, or obtained from, blood samples of athletes unable to sustain normal training levels, curves produced using leucocytes in, or obtained from, blood samples of individuals suffering from a viral or bacterial infection, and curves produced using leucocytes in, or obtained from, blood samples of normal, fit and healthy individuals.

4. A method according to claim 3, wherein the curves obtained using leucocytes in blood samples of individuals suffering from a viral or bacterial infection are blood samples from individuals suffering from viral infections of the respiratory tract.

5. A method according to any one of claims 1 to 4, wherein the blood sample is a sample of whole blood or of isolated blood cells.

6. A method according to any one of claims 1 to 5, wherein the luminescence reagent is the photoprotein derived from *Pholas dactylus* or a synthetic equivalent thereof.

7. A method according to either claim 1 or claim 2, wherein the individual is a non-human mammal.

8. A method according to claim 7, wherein the luminescence reagent is the photoprotein derived from *Pholas dactylus* or a synthetic equivalent thereof.

**Patentansprüche**

1. Verfahren zur Erkennung des Beginns oder des Vorhandenseins des Übertrainingssyndroms bei einem Individuum, wobei das Verfahren umfasst:

a) Inkontaktbringen von Leukozyten in oder erhalten aus einer vom Individuum bereitgestellten Blutprobe mit einem Lumineszenzreagens, das bei Reaktion mit einem Oxidationsmittel Licht emittiert;

b) Zugeben eines Aktivators zum Gemisch von Leukozyten und dem Lumineszenzreagens, um das NADPH-Oxidasesystem der Leukozyten zu stimulieren, wobei der Aktivator aus N-Formyl-methionyl-leucyl-phenylalanin und Thrombozytenaktivierungsfaktor ausgewählt ist;

c) kontinuierliches Überwachen und/oder Messen von Licht, das vom Lumineszenzreagens emittiert wird, über eine vorbestimmte Zeitdauer, die vor der Zugabe des Aktivators beginnt und danach endet, wobei eine Lichtemissionskurve erzeugt wird, welche das vom Lumineszenzreagens emittierte Licht über die vorbestimmte Zeitdauer zeigt;

d) Auswerten der Merkmale der Lichtemissionskurve zum Bestimmen des Beginns oder des Vorhandenseins des Übertrainingssyndroms beim Individuum durch Vergleich mit einer oder mehreren Standardsignaturkurven, und wobei das Bestimmen auf der Ähnlichkeit oder Unähnlichkeit basiert, die zwischen Merkmalen der Lichtemissionskurve und den Merkmalen der einen oder der mehreren Standardsignaturkurven auftritt.

2. Verfahren nach Anspruch 1, wobei die Merkmale der Lichtemissionskurve und die Merkmale der einen oder der mehreren Standardsignaturkurven aus Kurvenform, Intensität der Kurve und einer Kombination davon ausgewählt sind.

**3.** Verfahren nach Anspruch 1 oder 2, wobei die Lichtemissionskurve unter Verwendung von Leukozyten in oder erhalten aus einer einem Athleten entnommenen Blutprobe erzeugt wird, und wobei Standardsignaturkurven ausgewählt sind aus Kurven, die unter Verwendung von Leukozyten in oder erhalten aus Blutproben von Athleten erzeugt werden, von welchen klinisch bestätigt wurde, dass sie an übermäßiger oder Dauermüdigkeit leiden, Kurven, die unter Verwendung von Leukozyten in oder erhalten aus Blutproben von Athleten erzeugt werden, die an schlechter Wettkampfleistung leiden, Kurven, die unter Verwendung von Leukozyten in oder erhalten aus Blutproben von Athleten erzeugt werden, die nicht imstande sind, normale Trainingsniveaus aufrechtzuerhalten, Kurven, die unter Verwendung von Leukozyten in oder erhalten aus Blutproben von Individuen erzeugt werden, die an einer Virus- oder Bakterieninfektion leiden, und Kurven, die unter Verwendung von Leukozyten in oder erhalten aus Blutproben von normalen, fitten und gesunden Individuen erzeugt werden.

**4.** Verfahren nach Anspruch 3, wobei die Kurven, die unter Verwendung von Leukozyten in Blutproben von Individuen erzeugt werden, die an einer Virus- oder Bakterieninfektion leiden, Blutproben von Individuen sind, die an Virusinfektionen der Atemwege leiden.

**5.** Verfahren nach einem der Ansprüche 1 bis 4, wobei die Blutprobe eine Probe von Vollblut oder von isolierten Blutzellen ist.

**6.** Verfahren nach einem der Ansprüche 1 bis 5, wobei das Lumineszenzreagens das Photoprotein, das aus *Pholas dactylus* gewonnen wird, oder ein synthetisches Äquivalent davon ist.

**7.** Verfahren nach Anspruch 1 oder 2, wobei das Individuum ein nichtmenschlicher Säuger ist.

**8.** Verfahren nach Anspruch 7, wobei das Lumineszenzreagens das Photoprotein, das aus *Pholas dactylus* gewonnen wird, oder ein synthetisches Äquivalent davon ist.


**Revendications**

**1.** Procédé pour détecter l'apparition ou l'existence d'un syndrome de surentraînement chez un individu, lequel procédé consiste à

   a) mettre en contact des leucocytes dans, ou obtenu à partir de, un échantillon de sang fourni par l'individu avec un réactif de luminescence qui émet de la lumière par réaction avec un oxydant ;
   b) ajouter un activateur au mélange de leucocytes et au réactif de luminescence pour stimuler le système de NADPH oxydase des leucocytes, où l'activateur est choisi parmi de la N-formyl-méthionyl-leucyl-phénylalanine et un facteur d'activation plaquettaire ;
   c) suivre en continu et/ou mesurer la lumière émise par le réactif de luminescence sur une période temporelle prédéterminée commençant avant et se terminant après l'ajout de l'activateur, grâce à quoi une courbe d'émission de lumière est produite et illustre la lumière émise par le réactif de luminescence sur la période temporelle prédéterminée ;
   d) évaluer les caractéristiques de la courbe d'émission de lumière pour déterminer l'apparition ou l'existence d'un syndrome de surentraînement chez l'individu par comparaison avec une ou plusieurs courbes de signature standard et la détermination est basée sur la similarité ou la différence survenant entre les caractéristiques de la courbe d'émission de lumière et les caractéristiques de la ou des courbes de signature standard.

**2.** Procédé selon la revendication 1, où les caractéristiques de la courbe d'émission de lumière et les caractéristiques de la ou des courbes de signature standard sont sélectionnées à partir de la forme de la courbe, de l'intensité de la courbe et d'une combinaison de celles-ci.

**3.** Procédé selon l'une ou l'autre de la revendication 1 ou la revendication 2, où la courbe d'émission de lumière est produite en utilisant des leucocytes dans, ou obtenus à partir de, un échantillon de sang prélevé sur un athlète et où les courbes de signature standard sont sélectionnées à partir de courbes produites en utilisant des leucocytes dans, ou obtenus à partir, des échantillons de sang d'athlètes cliniquement confirmés comme souffrant d'une fatigue excessive ou persistante, de courbes produites en utilisant des leucocytes dans, ou obtenus à partir, des échantillons de sang d'athlètes souffrant de mauvaises performances en compétition, de courbes produites en utilisant des leucocytes dans, ou obtenus à partir, des échantillons de sang d'athlètes incapables de maintenir des niveaux d'entraînement normaux, de courbes produites en utilisant des leucocytes dans, ou obtenus à partir, des échantillons de sang d'individus souffrant d'une infection virale ou bactérienne, et de courbes produites en utilisant des leucocytes dans, ou obtenus à partir, des échantillons de sang d'individus normaux en bonne santé.

4. Procédé selon la revendication 3, où les courbes obtenues en utilisant des leucocytes dans des échantillons de sang d'individus souffrant d'une infection virale ou bactérienne sont des échantillons de sang provenant d'individus souffrant d'infections virales des voies respiratoires.

5. Procédé selon une quelconque des revendications 1 à 4, où l'échantillon de sang est un échantillon de sang entier ou de cellules sanguines isolées.

6. Procédé selon une quelconque des revendications 1 à 5, où le réactif de luminescence est la photoprotéine dérivée de Pholas dactylus ou un équivalent synthétique de celle-ci.

7. Procédé selon l'une ou l'autre de la revendication 1 ou la revendication 2, où l'individu est un mammifère non humain.

8. Procédé selon la revendication 7, où le réactif de luminescence est la photoprotéine dérivée de Pholas dactylus ou un équivalent synthétique de celle-ci.

Light emission curves from four different blood samples
labelled: A,B,C,D. All curves have different maximum light values

# FIG. 1

The light emission curves from figure 1 are now expressed as a percentage of maximal light

# FIG. 2

FIG. 3

FIG. 4

FIG. 5

FIG. 6